# EUROPEAN PATENT APPLICATION

(11) **EP 4 693 319 A1**
(43) Date of publication of application: **11.02.2026**
(21) Application number: 25785526.2
(22) Date of filing: 12.03.2025
(51) Int. Cl.: G16H 15/00

(54) **CLINICAL TRIAL REPORT GENERATION METHOD AND DEVICE FOR CLINICAL TRIAL DATA, AND MEDIUM**

(30) Priority: 08.04.2024 CN 202410411133
(71) Applicant: Iwe Technology (Beijing) Co., Ltd., Beijing 100022 (CN)
(72) Inventor: GUO, Mengjie, Beijing 100022 (CN)
(74) Representative: Huang, Liwei
(86) International application number: PCT/CN2025/082041
(87) International publication number: WO 2025/214046

(57) **Abstract**

The present invention discloses a method, device, and medium for generating clinical trial reports based on clinical trial data. The method includes: creating a clinical trial report generation project corresponding to clinical trial data to be processed through a clinical trial report generation platform, and assigning corresponding roles and permissions to relevant processing personnel of the clinical trial report generation project; constructing, by the statistical analysts, a report template to be reviewed based on a clinical trial design, and publishing the report template to be reviewed; performing online reviews, by the reviewers, on the report template to be reviewed, and adding online collaborative annotations, such that the statistical analysts update the report template to be reviewed to be a report template to be applied based on online collaborative annotations; annotating, by the annotators, the report template to be applied, such that the clinical trial report generation platform constructs a corresponding SAS program based on the annotations; and executing the SAS program to obtain a clinical trial report to be applied. Through the above method, the present invention efficiently achieves automatic generation of clinical trial analysis reports.

## Description

The present invention claims the priority of the Chinese patent application filed with the Chinese Patent Office on April 8, 2024, with an application number of "202410411133.1" and entitled "Method, Device and Medium for Generating Clinical Trial Reports Based on Clinical Trial Data", the contents of which are incorporated herein by reference in its entirety.

### TECHNICAL FIELD

The present invention relates to the technical field of analysis of medical research and development data, in particular to a method, device, and medium for generating clinical trial reports based on clinical trial data.

### BACKGROUND

The field of analysis of medical research and development data is a complex working field that integrates a plurality of disciplines, including medicine, statistics, and computer science. Having developed in China for over 20 years, the industry scale has been consistently in a phase of growth. However, there are very few specialized programs offered by domestic universities that align with this field, creating a demand for interdisciplinary talents. Furthermore, the vast majority of tasks still rely heavily on manual labor, resulting in a persistent talent shortage to this day. Analysis of medical research and development data has extremely high requirements for quality and an extremely low tolerance for errors. As required by industry practices, all calculation results typically require quality control through "independent dual programming", that is, two analysts independently write programs for calculations, and their results must be 100% consistent before approval is granted. Therefore, this field highly relies on personnel and requires a large number of working hours for programming, calculations, and quality control.

In terms of submission of clinical trial data, in response to the advent of an electronic submission era and following requirements of the European Union in 2014 that pharmaceutical companies must submit data in a format of eCTD (Electronic Common Technical Document), the U.S. FDA (Food and Drug Administration) also imposed a mandatory regulation in 2016. After nearly 20 years of development and upgrades, CDISC (Clinical Data Interchange Standards Consortium) has developed a plurality of modules to support clinical data standards: SEND/SDTM/ADaM/Define.xml. Accompanying these modules are clinical trial statistical reports created based on an SAP (Statistical Analysis Plan) and attached to these modules. The number of clinical trial reports varies depending on the complexity of projects, ranging from hundreds to thousands. Therefore, the above modules, together with clinical trials, have become the main and critical tasks for statistical analysis teams of pharmaceutical companies and CROs (Contract Research Organizations) to meet submission requirements. Therefore, how to efficiently achieve automated generation of clinical trial reports has become a technical problem to be urgently solved.

### SUMMARY

Embodiments of the present invention provide a method, device, and medium for generating clinical trial reports based on clinical trial data, to solve the following technical problem: how to efficiently achieve automated generation of clinical trial reports.

In a first aspect, the embodiments of the present invention provide a method for generating clinical trial reports based on clinical trial data. The method includes: creating a clinical trial report generation project corresponding to clinical trial data to be processed through a clinical trial report generation platform, and assigning corresponding roles and permissions to relevant processing personnel of the clinical trial report generation project; wherein the relevant processing personnel include: statistical analysts, reviewers, and annotators; constructing, by the statistical analysts, a report template to be reviewed based on a clinical trial design, and publishing the report template to be reviewed; performing online reviews, by the reviewers, on the report template to be reviewed, and adding online collaborative annotations, such that the statistical analysts update the report template to be reviewed based on the online collaborative annotations and obtain a report template to be applied; annotating, by the annotators, the report template to be applied, such that the clinical trial report generation platform constructs a corresponding SAS (Statistical Analysis System) program based on the annotations; and executing the SAS program to obtain a clinical trial report to be applied.

In one embodiment of the present invention, the constructing the report template to be reviewed based on the clinical trial design includes: determining keywords to be searched based on the clinical trial design, and reading a report style knowledge base based on the keywords to be searched to determine whether there are relevant templates with a matching degree greater than a preset threshold; when it is determined that there are relevant templates with a matching degree greater than the preset threshold, adjusting the relevant templates based on the clinical trial design to obtain the report template to be reviewed; and when it is determined that there are relevant templates with a matching degree less than the preset threshold, generating an empty template, and constructing the report template to be reviewed based on the clinical trial design.

In one embodiment of the present invention, the method further includes: marking different field sections among several similar report templates to be reviewed or several similar report templates to be applied; determining any one of the several report templates to be reviewed or the several report templates to be applied as a report template to be operated, and determining other report templates as synchronous report templates; and when the report template to be operated is modified, triggering an update component corresponding to the synchronous report templates to complete the update of the corresponding synchronous report templates.

In one embodiment of the present invention, the annotating the report template to be applied includes: determining parameter report fields to be annotated in the report template to be applied, and performing programmatic or natural language markup annotations on the parameter report fields; triggering a logic verification function to perform logic verification on the programmatic or natural language markup annotations, so as to determine whether the programmatic or natural language markup annotations conform to writing specifications; and when it is determined that the programmatic or natural language markup annotations conform to the writing specifications, completing the annotation of the report template to be applied.

In one embodiment of the present invention, the constructing, by the clinical trial report generation platform, a corresponding SAS program based on the annotations includes: parsing the annotations by the clinical trial report generation platform through cross-platform computer programming languages and SAS, so as to convert the annotations into SAS core calculation macro programs and parameters corresponding to automatic filling macros; and summarizing the SAS core calculation macro programs and the parameters corresponding to the automatic filling macros to obtain a corresponding SAS program.

In one embodiment of the present invention, after the parsing the annotations by the clinical trial report generation platform through cross-platform computer programming languages and SAS, the method further includes: parsing the clinical trial data to be processed to parse the clinical trial data to be processed into variable units corresponding to filling parameters; and summarizing the annotations and the variable units to obtain an ADaM (Analysis Data Model) analysis dataset derivation specification document.

In one embodiment of the present invention, after executing the SAS program to obtain the clinical trial report to be applied, the method further includes: storing the clinical trial report to be applied and SAS result datasets generated by executing the SAS program.

In one embodiment of the present invention, the method further includes: when the reviewers perform online reviews on the report template to be reviewed and the clinical trial report to be applied, and add online collaborative annotations, generating in real time and storing communication logs.

In a second aspect, the embodiments of the present invention further provide a device for generating clinical trial reports based on clinical trial data. The device includes: at least one processor; and a memory communicatively connected to the at least one processor; wherein the memory stores instructions executable by the at least one processor, and the instructions are executed by the at least one processor, such that the at least one processor is configured to: create a clinical trial report generation project corresponding to clinical trial data to be processed through a clinical trial report generation platform, and assign corresponding roles and permissions to relevant processing personnel of the clinical trial report generation project; wherein the relevant processing personnel include: statistical analysts, reviewers, and annotators; construct, by the statistical analysts, a report template to be reviewed based on a clinical trial design, and publish the report template to be reviewed; perform online reviews, by the reviewers, on the report template to be reviewed, and add online collaborative annotations, such that the statistical analysts update the report template to be reviewed based on the online collaborative annotations and obtain a report template to be applied; annotate, by the annotators, the report template to be applied, such that the clinical trial report generation platform constructs a corresponding SAS program based on the annotations; and execute the SAS program to obtain a clinical trial report to be applied.

In a third aspect, the embodiments of the present invention further provide non-volatile computer storage medium for generating clinical trial reports based on clinical trial data, wherein the non-volatile computer storage medium stores computer-executable instructions, and the computer-executable instructions are configured to: create a clinical trial report generation project corresponding to clinical trial data to be processed through a clinical trial report generation platform, and assign corresponding roles and permissions to relevant processing personnel of the clinical trial report generation project; wherein the relevant processing personnel include: statistical analysts, reviewers, and annotators; construct, by the statistical analysts, a report template to be reviewed based on a clinical trial design, and publish the report template to be reviewed; perform online reviews, by the reviewers, on the report template to be reviewed, and add online collaborative annotations, such that the statistical analysts update the report template to be reviewed based on the online collaborative annotations and obtain a report template to be applied; annotate, by the annotators, the report template to be applied, such that the clinical trial report generation platform constructs a corresponding SAS program based on the annotations; and execute the SAS program to obtain a clinical trial report to be applied.

The method, device, and medium for generating clinical trial reports based on clinical trial data provided in the embodiments of the present invention have strong online collaboration capabilities, greatly reduce communication costs during review and update processes, make the project status clear at a glance, eliminate the need for repeated communication and confirmation, and retain all communication records that can be exported for filing. The novel WYSIWYG (What You See Is What You Get) method used in the present invention eliminates cognitive inconsistencies between the reviewers and programmers, and the final clinical trial reports will be consistent in style with the report templates seen by the reviewers. In the present invention, after the annotations are parsed and summarized, machines generate SAS programs and ADaM analysis dataset derivation specification documents simultaneously. This breaks an original workflow of first writing dataset specification documents, generating datasets, and then writing clinical trial programs, and adopts a result-oriented approach. The ADaM analysis dataset is configured to conduct what is required by subsequent analysis, and the relationship between the two modules is one-to-one correspondence, thereby avoiding creating a large number of redundant variables that are not used in a backend and preventing situations where variables expected to be used in clinical trial reports are found to have not been created in the dataset earlier only after executing the SAS programs. In the present invention, machines replace traditional manual labor to automatically generate SAS programs, featuring high efficiency, stronger control over details (including formats and layouts), lower risks of rework or revision, reduced repetitive work, and guaranteed consistency and quality. In the present invention, by storing the clinical trial reports to be applied and SAS result datasets generated by executing the SAS program, comparison between different versions of the clinical trial reports accurately locates the positions where changes have occurred, results of previous versions are displayed, and the problem of misalignment is solved.

### BRIEF DESCRIPTION OF THE DRAWINGS

The drawings described herein are provided to further understand the present invention and constitute a part of the present invention. The schematic embodiments of the present invention and their descriptions are used to explain the present invention, and do not constitute an improper limitation of the present invention. In the drawings:
Fig. 1 is a flowchart of a method for generating clinical trial reports based on clinical trial data provided in an embodiment of the present invention;
Fig. 2 is a schematic diagram of an internal structure of a device for generating clinical trial reports based on clinical trial data provided in an embodiment of the present invention.

### DETAILED DESCRIPTION OF THE EMBODIMENTS

To make the objectives, technical solutions, and advantages of the present invention clearer, the technical solutions of the present invention will be clearly and completely described below in conjunction with specific embodiments of the present invention and the corresponding accompanying drawings. Obviously, the described embodiments are only a part but not all of the embodiments of the present invention. Based on the embodiments in the present invention, all the other embodiments obtained by those skilled in the art without any creative effort shall fall within the protection scope of the present invention.

Statistical analysis is performed on the data collected according to clinical research protocols. Simply speaking, the most fundamental task is to use SAS (a professional statistical analysis software recommended in the life sciences field, with all its calculation modules certified) to generate datasets (SDTM (Study Data Tabulation Model), ADAM) that meet CDISC standards as required by regulatory authorities, as well as hundreds of clinical trial reports for the preparation and submission of clinical study reports (CSR).

Before submission, designing the formats of clinical trial reports based on the SAP and generating statistical analysis reports is a key task for statistical analysts. Statistical analysis teams in pharmaceutical companies and CROs need to invest significant manpower and resources.

The following problems exist.
1. Report complexity: Clinical trial reports usually require frequent revisions to contents and formats based on different clinical trials or different trial phases, and contain a large amount of data and statistical analysis results. During the report preparation and review processes, multiple generations and updates may be needed based on actual data; in addition, revisions to datasets may be required retroactively if the earlier dataset preparation is inadequate, such that the finalization process of reports becomes complex and the statistical analysis teams need to invest considerable time and effort.
2. Data quality problems: Data quality is crucial to the quality and accuracy of clinical trial reports. However, in practical operations, problems such as data errors, data missing, and data inconsistencies may slow down the report preparation process and even affect the final results.
3. Communication and coordination: The preparation of clinical trial reports involves a plurality of departments and teams, including data management, statistical analysis, and medical writing. Therefore, communication and coordination are of vital importance. Improper communication and coordination may lead to inconsistencies between report design and requirements, and excessive resource consumption, and may further affect report quality.
4. Time pressure: Clinical research usually has strict time constraints. Therefore, the statistical analysis teams need to complete the preparation of clinical trial reports within a short period, thereby leading to overtime work of team members and increased work pressure.
5. Compliance and quality control: Clinical trial reports are an important part of clinical study reports and should meet the requirements of relevant regulations and standards.

Therefore, statistical analysis teams need to focus on compliance and quality control during the report preparation process to ensure accuracy and reliability of the reports.

The embodiments of the present invention provide a method, device, and medium for generating clinical trial reports based on clinical trial data, to solve the following technical problem: how to efficiently realize automated generation of clinical trial analysis reports.

The technical solutions proposed in the embodiments of the present invention will be described in detail below with reference to the accompanying drawings.

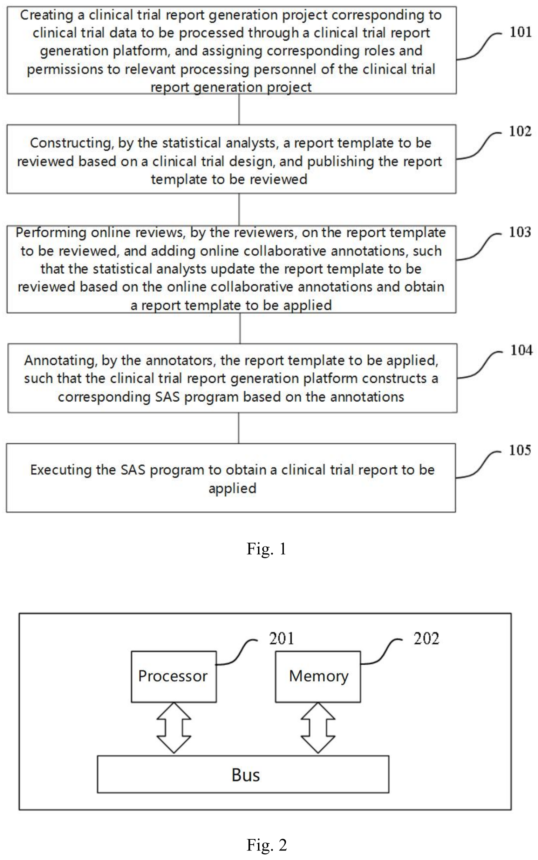

Fig. 1 is a flowchart of a method for generating clinical trial reports based on clinical trial data provided in an embodiment of the present invention. As shown in Fig. 1, the method for generating clinical trial reports based on clinical trial data provided in the embodiment of the present invention specifically includes the following steps:
Step 101: creating a clinical trial report generation project corresponding to clinical trial data to be processed through a clinical trial report generation platform, and assigning corresponding roles and permissions to relevant processing personnel of the clinical trial report generation project.

In one embodiment of the present invention, to implement the method for generating clinical trial reports based on clinical trial data, the user first needs to create a clinical trial report generation project corresponding to the clinical trial data to be processed through a clinical trial report generation platform. After the project creation application is approved, corresponding roles and permissions are assigned to the relevant processing personnel of the clinical trial report generation project. Different roles have different task permissions, to prevent other personnel from making incorrect operations on documents.

It should be noted that the relevant processing personnel include: statistical analysts, reviewers, and annotators.

Step 102: constructing, by the statistical analysts, a report template to be reviewed based on a clinical trial design, and publishing the report template to be reviewed.

In one embodiment of the present invention, after creating a clinical trial report generation project and assigning corresponding roles and permissions to the relevant processing personnel of the clinical trial report generation project, the statistical analysts construct a report template to be reviewed based on the clinical trial design.

Based on the clinical trial design, the keywords to be searched are determined, and the report style knowledge base is read based on the keywords to be searched to determine whether there are relevant templates with a matching degree greater than a preset threshold; if it is determined that there are relevant templates with a matching degree greater than the preset threshold, the relevant templates are adjusted based on the clinical trial design to obtain the report template to be reviewed; if it is determined that there are relevant templates with a matching degree less than the preset threshold, an empty template is generated, and the report template to be reviewed is constructed based on the clinical trial design.

Based on the novel WYSIWYG (What You See Is What You Get) method, all the text and formats in the report template to be reviewed will be parsed by cross-platform computer programming languages and SAS. Such text and formats include titles, footnotes, column headers, display text of each paragraph, blank lines between paragraphs, presentation of decimal places, and so on. Eventually, all these elements will be fully presented in the final clinical trial reports. The platform is also able to export clinical trial reports in other formats (such as Word and PDF) to meet different needs such as project team filing.

Further, the statistical analysts release the report template to be reviewed.

Step 103: performing online reviews, by the reviewers, on the report template to be reviewed, and adding online collaborative annotations, such that the statistical analysts update the report template to be reviewed based on the online collaborative annotations and obtain a report template to be applied.

In one embodiment of the present invention, after the statistical analysts release the report template to be reviewed, the reviewers perform online reviews on the report template to be reviewed and add online collaborative annotations.

The reviewers perform online reviews on the report template to be reviewed and add comments or revision suggestions in real time. The status will be updated synchronously in real time, and emails and Lark messages are sent to relevant personnel in real time. After receiving the comments, the statistical analysts can update or respond based on the online collaborative annotations. There is no need for back-and-forth communication methods such as email exchanges. This online collaboration method greatly reduces the communication costs among staff.

In one embodiment of the present invention, when the reviewers perform online reviews on the report template to be reviewed and add online collaborative annotations, communication logs are generated in real time and saved to avoid unnecessary disputes.

In one embodiment of the present invention, to achieve batch and rapid update of report templates, when the report template to be applied is generated in the present invention, different field sections among several similar report templates to be reviewed or several report templates to be applied are also marked; any one of the several report templates to be reviewed or the several report templates to be applied is determined as a report template to be operated, and the other report templates are determined as synchronous report templates; when the report template to be operated is modified, an update component corresponding to the synchronous report templates is triggered to complete the update of the corresponding synchronous report templates.

Step 104: annotating, by the annotators, the report template to be applied, such that the clinical trial report generation platform constructs a corresponding SAS program based on the annotations.

In one embodiment of the present invention, after obtaining the report template to be applied, the annotators first annotate the report template to be applied.

Parameter report fields to be annotated are determined in the report template to be applied, and programmatic or natural language markup annotations are performed on the parameter report fields; a logic verification function is triggered to perform logic verification on the programmatic or natural language markup annotations to determine whether the programmatic or natural language markup annotations conform to writing specifications; if it is determined that the programmatic or natural language markup annotations conform to the writing specifications, the annotation of the report template to be applied is completed.

Further, the clinical trial report generation platform constructs the corresponding SAS program based on the annotations.

The clinical trial report generation platform parses the annotations through cross-platform computer programming languages and SAS to convert the annotations into SAS core calculation macro programs and parameters corresponding to automatic filling macros; the SAS core calculation macro programs and the parameters corresponding to automatic filling macros are summarized to obtain the corresponding SAS program.

It should be noted that in specific application, R language and cross-platform computer programming languages are also used to replace SAS for core calculations related to statistical analysis.

In one embodiment of the present invention, after the clinical trial report generation platform parses the annotations through cross-platform computer programming languages and SAS, the method further includes: parsing the clinical trial data to be processed to parse the clinical trial data to be processed into variable units corresponding to filling parameters; and summarizing the annotations and the variable units to obtain an ADaM analysis dataset derivation specification document.

It should be noted that after annotation and parsing, the data will also be parsed into variable units by the backend and summarized with all the annotations within the same project. Finally, these summarized results will be automatically generated into an ADaM analysis dataset derivation specification document in accordance with CDISC standards, and the document includes all datasets and variable information required for generating clinical trial reports. There is no need to manually create blank specification documents and fill the documents in one variable at a time.

Step 105: executing the SAS program to obtain a clinical trial report to be applied.

In one embodiment of the present invention, after constructing the corresponding SAS program, the SAS program is executed to obtain the clinical trial report to be applied.

Further, after executing the SAS program to obtain the clinical trial report to be applied, the clinical trial report to be applied and SAS result datasets generated by executing the SAS program are stored.

It should be noted that the SAS result datasets and clinical trial reports of the analysis reports generated each time the SAS program is executed will be saved in a designated folder. This facilitates storage of different versions and allows version comparison. Differences (additions, deletions, and modifications) will be intuitively displayed in the clinical trial report and marked with color highlighting.

Modification: modification represents that there are changes in results between different versions, marked in red with the previous results displayed. Addition: new result rows are added compared with the comparison version, marked in green.

Deletion: result rows are added compared with the comparison version, marked in gray and placed at the end.

The technology used in this function is that independent Key variables are defined for clinical trial report results (each cell of the clinical trial report stores row and column coordinate information, text information, i.e., input dataset, variable name, variable value or label, conditions, etc.). These Key variables are used to identify each row, avoiding the problem that comparison results are difficult to recognize due to misalignment problems during version comparison.

The above are method embodiments proposed in the present invention. Based on the same inventive concept, the embodiments of the present invention also provide a device for generating clinical trial reports based on clinical trial data, and the structure is shown in Fig. 2.

Fig. 2 is a schematic diagram of an internal structure of a device for generating clinical trial reports based on clinical trial data provided in an embodiment of the present invention. As shown in Fig. 2, the device includes:
at least one processor 201;
and a memory 202 communicatively connected to the at least one processor;
wherein the memory 202 stores instructions executable by the at least one processor, and the instructions are executed by the at least one processor 201, such that the at least one processor 201 is configured to:
   create a clinical trial report generation project corresponding to clinical trial data to be processed through a clinical trial report generation platform, and assign corresponding roles and permissions to relevant processing personnel of the clinical trial report generation project; wherein the relevant processing personnel include: statistical analysts, reviewers, and annotators;
   construct, by the statistical analysts, a report template to be reviewed based on a clinical trial design, and publish the report template to be reviewed;
   perform online reviews, by the reviewers, on the report template to be reviewed, and add online collaborative annotations, such that the statistical analysts update the report template to be reviewed based on the online collaborative annotations and obtain a report template to be applied;
   annotate, by the annotators, the report template to be applied, such that the clinical trial report generation platform constructs a corresponding SAS program based on the annotations; and
   execute the SAS program to obtain a clinical trial report to be applied.

Some embodiments of the present invention provide a non-volatile computer storage medium for generating clinical trial reports based on clinical trial data corresponding to Fig. 1, the non-volatile computer storage medium stores computer-executable instructions, and the computer-executable instructions are configured to:
create a clinical trial report generation project corresponding to clinical trial data to be processed through a clinical trial report generation platform, and assign corresponding roles and permissions to relevant processing personnel of the clinical trial report generation project; wherein the relevant processing personnel include: statistical analysts, reviewers, and annotators;
construct, by the statistical analysts, a report template to be reviewed based on the clinical trial design, and publish the report template to be reviewed;
perform online reviews, by the reviewers, on the report template to be reviewed, and add online collaborative annotations, such that the statistical analysts update the report template to be reviewed based on the online collaborative annotations and obtain a report template to be applied;
annotate, by the annotators, the report template to be applied, such that the clinical trial report generation platform constructs a corresponding SAS program based on the annotations; and
execute the SAS program to obtain a clinical trial report to be applied.

Each embodiment in the present invention is described in a progressive manner. For the same or similar parts between the various embodiments, reference may be made to each other, and each embodiment focuses on explaining the differences from other embodiments. In particular, as the embodiments of the IoT device and the medium are basically similar to the method embodiment, their descriptions are relatively simple, and for relevant parts, reference may be made to the partial explanations of the method embodiment.

The systems and media provided in the embodiments of the present invention are in one-to-one correspondence with the methods. Therefore, the systems and media also have beneficial technical effects similar to those of their corresponding methods. Since the beneficial technical effects of the methods have been described in detail above, the beneficial technical effects of the systems and media will not be repeated herein.

Those skilled in the art should understand that the embodiments of the present invention can be provided as a method, a system, or a computer program product. Therefore, the present invention can adopt the form of an entirely hardware embodiment, an entirely software embodiment, or an embodiment combining software and hardware aspects. Furthermore, the present invention can adopt the form of a computer program product implemented on one or more computer-usable storage media (including but not limited to disc storage devices, CD-ROMs, optical storage devices, etc.) that contain computer-usable program codes.

The present invention is described with reference to flowcharts and/or block diagrams of methods, devices (systems), and computer program products according to the embodiments of the present invention. It should be understood that each process and/or block in the flowcharts and/or block diagrams, as well as combinations of processes and/or blocks in the flowcharts and/or block diagrams, can be implemented by computer program instructions. These computer program instructions may be provided to the processor of a general-purpose computer, a special-purpose computer, an embedded processor, or other programmable data processing devices to produce a machine, such that the instructions executed by the processor of the computer or other programmable data processing devices generate an apparatus for implementing the functions specified in one or more processes of the flowchart and/or one or more blocks of the block diagram.

These computer program instructions may also be stored in a computer-readable memory that can direct a computer or other programmable data processing devices to operate in a specific manner, such that the instructions stored in the computer-readable memory produce an article of manufacture including an instruction apparatus, and the instruction apparatus implements the functions specified in one or more processes of the flowchart and/or one or more blocks of the block diagram.

These computer program instructions may also be loaded onto a computer or other programmable data processing devices, such that a series of operational steps are performed on the computer or other programmable devices to produce a computer-implemented process, and the instructions executed on the computer or other programmable devices provide steps for implementing the functions specified in one or more processes of the flowchart and/or one or more blocks of the block diagram.

In a typical configuration, a computing device includes one or more processors (CPUs (Central Processing Units)), input/output interfaces, network interfaces, and a memory.

The memory may include a non-permanent storage device in computer-readable media, in the form of a random access memory (RAM) and/or a non-volatile memory, such as a read-only memory (ROM) or a flash memory (flash RAM). The memory is an example of the computer-readable medium.

The computer-readable media include both permanent and non-permanent, removable and non-removable media, and information can be stored by any method or technology. The information may be computer-readable instructions, data structures, program modules, or other data. Examples of computer storage media include, but are not limited to, phase-change memories (PRAMs), static random access memories (SRAMs), dynamic random access memories (DRAMs), other types of random access memories (RAMs), read-only memories (ROMs), electrically erasable programmable read-only memories (EEPROMs), flash memories or other memory technologies, compact disc read-only memories (CD-ROMs), digital versatile discs (DVDs) or other optical storage devices, magnetic tape cartridges, magnetic tapes or magnetic disk storage devices or other magnetic storage devices, or any other non-transmission media that can be used to store information accessible by a computing device. As defined in this document, computer-readable media do not include transitory computer-readable media, such as modulated data signals and carrier waves.

It should also be noted that the terms "include", "comprise" or any other variations thereof are intended to cover a non-exclusive inclusion, such that a process, method, article, or device that includes a list of elements not only includes those elements, but also includes other elements not explicitly listed, or elements inherent to such a process, method, article, or device. Without further limitations, an element defined by the phrase "comprises a..." does not exclude the presence of additional identical elements in the process, method, article, or device that includes the element.

The above descriptions are merely embodiments of the present invention and are not intended to limit the present invention. For those skilled in the art, various modifications and changes may be made to the present invention. Any modification, equivalent substitution, improvement and the like made within the spirit and principle of the present invention shall be included in the scope of the claims of the present invention.

## Claims

1. A method for generating a clinical trial report based on clinical trial data, wherein the method comprises:
creating, by a clinical trial report generation platform, a clinical trial report generation project corresponding to clinical trial data to be processed, and assigning corresponding roles and permissions to relevant processing personnel of the clinical trial report generation project; wherein the relevant processing personnel comprise: a statistical analyst, a reviewer, and an annotator;
constructing, by the statistical analyst, a report template to be reviewed based on a clinical trial design, and publishing the report template to be reviewed;
performing an online review, by the reviewer, on the report template to be reviewed, and adding online collaborative annotations, such that the statistical analyst updates the report template to be reviewed based on the online collaborative annotations, so as to obtain a report template to be applied;
annotating, by the annotator, the report template to be applied, such that the clinical trial report generation platform constructs a corresponding SAS (Statistical Analysis System) program based on the annotations; and
executing the SAS program to obtain a clinical trial report to be applied.

2. The method for generating the clinical trial report based on clinical trial data according to claim 1, wherein the constructing the report template to be reviewed based on the clinical trial design comprises:
determining keywords to be searched based on the clinical trial design, and reading a report style knowledge base based on the keywords to be searched to determine whether there is a relevant template with a matching degree greater than a preset threshold;
when it is determined that there is relevant template with a matching degree greater than the preset threshold, adjusting the relevant template based on the clinical trial design to obtain the report template to be reviewed; and
when it is determined that there are only templates with matching degree less than the preset threshold, generating an empty template, and constructing the report template to be reviewed based on the clinical trial design.

3. The method for generating the clinical trial report based on clinical trial data according to claim 1, wherein the method further comprises:
marking different field sections among several similar report templates to be reviewed or marking several similar report templates to be applied;
determining any one of the several report templates to be reviewed or the several report templates to be applied as a report template to be operated, and determining other report templates as synchronous report templates; and
when the report template to be operated is modified, triggering an update component corresponding to the synchronous report templates to complete the update of the corresponding synchronous report templates.

4. The method for generating the clinical trial report based on clinical trial data according to claim 1, wherein the annotating the report template to be applied comprises:
determining parameter report fields to be annotated in the report template to be applied, and performing programmatic or natural language markup annotations on the parameter report fields;
triggering a logic verification function to perform logic verification on the programmatic or natural language markup annotations, so as to determine whether the programmatic or natural language markup annotations conform to writing specifications; and
when it is determined that the programmatic or natural language markup annotations conform to the writing specifications, completing the annotation of the report template to be applied.

5. The method for generating the clinical trial report based on clinical trial data according to claim 1, wherein the clinical trial report generation platform constructs a corresponding SAS program based on the annotations comprises:
parsing the annotations by the clinical trial report generation platform through cross-platform computer programming languages and SAS, so as to convert the annotations into SAS core calculation macro programs and parameters corresponding to automatic filling macros; and
summarizing the SAS core calculation macro programs and the parameters corresponding to the automatic filling macros to obtain a corresponding SAS program.

6. The method for generating the clinical trial report based on clinical trial data according to claim 5, wherein after the parsing the annotations by the clinical trial report generation platform through cross-platform computer programming languages and SAS, the method further comprises:
parsing the clinical trial data to be processed to parse the clinical trial data to be processed into variable units corresponding to filling parameters; and
summarizing the annotations and the variable units to obtain an ADaM (Analysis Data Model) analysis dataset derivation specification document.

7. The method for generating the clinical trial report based on clinical trial data according to claim 1, wherein after the executing the SAS program to obtain the clinical trial report to be applied, the method further comprises:
storing the clinical trial report to be applied and SAS result datasets generated by executing the SAS program.

8. The method for generating the clinical trial report based on clinical trial data according to claim 1, wherein the method further comprises:
when the reviewer performs the online review on the report template to be reviewed and the clinical trial report to be applied, and adds online collaborative annotations, generating in real time communication logs and storing the communication logs.

9. A device for generating the clinical trial report based on clinical trial data, wherein the device comprises:
at least one processor;
and a memory communicatively connected to the at least one processor;
wherein the memory stores instructions executable by the at least one processor, and when the instructions are executed by the at least one processor, the at least one processor is configured to:
create, by a clinical trial report generation platform, a clinical trial report generation project corresponding to clinical trial data to be processed , and assign corresponding roles and permissions to relevant processing personnel of the clinical trial report generation project; wherein the relevant processing personnel comprise: a statistical analyst, a reviewer, and an annotator;
construct, by the statistical analyst, a report template to be reviewed based on a clinical trial design, and publish the report template to be reviewed;
perform an online review, by the reviewer, on the report template to be reviewed, and add online collaborative annotations, such that the statistical analyst updates the report template to be reviewed based on the online collaborative annotations, so as to obtain a report template to be applied;
annotate, by the annotator, the report template to be applied, such that the clinical trial report generation platform constructs a corresponding SAS program based on the annotations; and
execute the SAS program to obtain a clinical trial report to be applied.

10. A non-volatile computer storage medium for generating a clinical trial report based on clinical trial data, wherein the non-volatile computer storage medium stores computer-executable instructions, and the computer-executable instructions are configured to:
create, by a clinical trial report generation platform, a clinical trial report generation project corresponding to clinical trial data to be processed , and assign corresponding roles and permissions to relevant processing personnel of the clinical trial report generation project; wherein the relevant processing personnel comprise: a statistical analyst, a reviewer, and an annotator;
construct, by the statistical analyst, a report template to be reviewed based on a clinical trial design, and publish the report template to be reviewed;
perform an online review, by the reviewer, on the report template to be reviewed, and add online collaborative annotations, such that the statistical analyst updates the report template to be reviewed based on the online collaborative annotations, so as to obtain a report template to be applied;
annotate, by the annotator, the report template to be applied, such that the clinical trial report generation platform constructs a corresponding SAS program based on the annotations; and
execute the SAS program to obtain a clinical trial report to be applied.
